# EUROPÄISCHE PATENTANMELDUNG

(11) **EP 3 476 407 A1**
(43) Veröffentlichungstag der Anmeldung: **01.05.2019**
(21) Anmeldenummer: 18189698.6
(22) Anmeldetag: 20.08.2018
(51) Int. Cl.: A61L 9/12

(54) **BEDUFTUNGSVORRICHTUNG**

(30) Priorität: 20.09.2017 DE 202017105689 U
(71) Anmelder: Florek, Andreas, 5430 Wettingen (CH); Domoradzki, Andreas, 6020 Innsbruck (AT)
(72) Erfinder: Florek, Andreas, 5430 Wettingen (CH); Domoradzki, Andreas, 6020 Innsbruck (AT)
(74) Vertreter: Patentanwälte Behrmann Wagner PartG mbB

(57) **Zusammenfassung**

Die Erfindung betrifft eine Beduftungsvorrichtung (1) zum Abgeben eines Duftes in einen Raum, mit einem Vorratsbehälter (3) für zu verdunstende Flüssigkeit sowie mit einem mit dem Vorratsbehälter (3) über mindestens eine Entleerungsöffnung (7) in fluidleitender Verbindung stehenden, eine Verdunstungsoberfläche anbietenden Verdunstungsmaterial (11), insbesondere Filz, Karton, Holz oder Schwammmaterial umfassenden Verdunstungseinrichtung (8), wobei der Vorratsbehälter (3) eine von der mindestens einen Entleerungsöffnung (7) separate Befüllöffnung (5) zum individuellen Befüllen aufweist, die über dieser zugeordnete Verschlussmittel (6), bevorzugt selbsttätig, nach einem Befüllvorgang, bevorzugt reversibel, verschließbar ist. Erfindungsgemäß ist vorgesehen, dass die Verschlussmittel (6) ein selbsttätig schließendes Rückschlagventil (6.1) umfassen.

## Beschreibung

Die Erfindung betrifft eine Beduftungsvorrichtung zum Abgeben eines Duftes in einen Raum gemäß dem Oberbegriff des Anspruchs 1, mit einem Vorratsbehälter für zu verdunstende Flüssigkeit sowie mit einer mit dem Vorratsbehälter über mindestens eine Entleerungsöffnung (des Vorratsbehälters) in fluidleitender Verbindung stehende, eine Verdundungsoberfläche anbietendes Verdunstungsmaterial, wie beispielsweise Filz, Karton, Holz oder Schwammmaterial umfassende, Verdunstungseinrichtung.

Beduftungseinrichtungen für Räume sind in unterschiedlichen Ausgestaltungen bekannt. So ist beispielsweise in der CH 705 826 B1 eine Beduftungsvorrichtung zum Abgeben eines Duftes in einen Raum beschrieben, die zwei relativ zueinander verschiebbare Gehäuseteile aufweist, in denen ein Filzmaterial angeordnet ist. Durch Relativverschieben der Gehäuseteile werden Verdunstungsöffnungen im Gehäuse geöffnet und geschlossen.

Daneben sind vergleichsweise einfach aufgebaute Beduftungsvorrichtungen bekannt geworden, umfassend ein meist kolbenförmiges Glas, durch dessen Einfüllöffnung Holzstäbe herausragen, die eine Verdunstungsoberfläche anbieten, wobei über die Anzahl der Holzstäbe die Beduftungsintensität reguliert bzw. eingestellt werden kann.

In der Regel werden bekannte Beduftungsvorrichtungen mit vom Hersteller definierten Beduftungsflüssigkeiten ausgeliefert. Es besteht jedoch das Bedürfnis nach einer individuellen Raumbeduftung, beispielsweise mit einem Parfüm der Hausherrin oder des Hausherrn. Ausgehend von dem vorgenannten Stand der Technik liegt der Erfindung daher die Aufgabe zugrunde, eine alternative, verbesserte Beduftungsvorrichtung zum Beduften von Räumen anzugeben, mit welcher die Räume mit individuellen Duftstoffen und/oder Funktionsdüften, beispielsweise Moskitos beduftbar sind.

Diese Aufgabe wird mit einer Beduftungsvorrichtung mit den Merkmalen des Anspruchs 1 gelöst, d.h. zunächst gemäß einer gattungsgemäßen Beduftungsvorrichtung , bei der der Vorratsbehälter (zur Aufnahme einer zu verdunstenden, duftenden Flüssigkeit) zusätzlich zu der mindestens einen Entleerungsöffnung, über die zu verdunstende Flüssigkeit aus dem Vorratsbehälter in die Verdunstungseinrichtung bzw. zu dem Verdunstungsmaterial strömen kann, mindestens eine weitere bzw. von der Entlüftungsöffnung separate, insbesondere von dieser beabstandete Befüllöffnung zum individuellen Befüllen des Vorratsbehälters mit einer individuellen Beduftungsflüssigkeit aufweist, wobei erfindungsgemäß der Befüllöffnung Verschlussmittel der Beduftungsvorrichtung zugeordnet sind, mit der die Befüllöffnung nach einem Befüllvorgang verschließbar ist. Dabei ist es besonders bevorzugt, wenn die Verschlussmittel derart ausgebildet sind, dass diese die Befüllöffnung nach einem Füllvorgang selbsttätig, d.h. ohne zutun des Benuters bzw. Befüllers verschließen. Ganz besonders bevorzugt sind die Verschlussmittel derart ausgebildet, dass diese die Befüllöffnung reversibel verschließen, d.h. derart ausgebildet sind, dass die Befüllöffnung mehrfach öffen- und verschließbar ist, um somit die Beduftungsvorrichtung nicht nur einmal, sondern mehrfach benutzen bzw. wieder befüllen zu können.

Vorteilhafte Ausgestaltungen der Erfindung sind in den Unteransprüchen angegeben. In den Rahmen der Erfindung fallen sämtliche Kombinationen aus zumindest zwei von in der Beschreibung, den Ansprüche und/oder den Figuren offenbarten Merkmalen.

Der Erfindung liegt der Gedanke zugrunde, bei der einen Vorratsbehälter für zu verdunstende (Duft-)Flüssigkeit aufweisenden Beduftungsvorrichtung, dem Vorratsbehälter neben der mindestens einen, insbesondere den mehreren Entleerungsöffnungen, über die zu verdunstende Flüssigkeit aus dem Vorratsbehälter zu der Verdunstungseinrichtung gelangen kann, eine verschließbare Befüllöffnung zuzuordnen, über die ein Benutzer den Vorratsbehälter mit einem individuellen Duft bzw. einer individuellen, zu verdunstenden (Duft-)Flüssigkeit befüllen kann. Zum Verschließen der Befüllöffnung nach einem Befüllvorgang umfasst die Beduftungsvorrichtung der Befüllöffnung zugeordnete Verschlussmittel. Im Hinblick auf deren konkrete Ausgestaltung gibt es unterschiedliche Möglichkeiten. Im einfachsten Fall handelt es sich um manuell bedienbare Verschlussmittel, die nach einem Befüllvorgang manuell vom Befüller bedient bzw. betätigt werden müssen, beispielsweise in Form eines Schraubverschlusses. Bevorzugt ist jedoch auch eine Ausführungsform, bei der die Verschlussmittel die Befüllöffnung nach einem Befüllvorgang selbsttätig verschließend ausgebildet sind, um insofern eine besonders komfortable Befüllung bzw. Bedienung zu ermöglichen und darüber hinaus eine unmittelbare bzw. schnelle Verschließung der Befüllöffnung zu gewährleisten, ohne einen spezifischen, bzw. eigenen, manuellen Verschließeingriff der Befüllperson notwendig zu machen. Hierdurch reduziert sich die Gefahr eines Auslaufens von in der Regel intensiv riechender bzw. duftender Flüssigkeit. Wie eingangs bereits erwähnt ist es bevorzugt, wenn die Verschlussmittel mehrfach nutzbar ausgebildet sind, d.h. dass mittels der Verschlussmittel die Befüllöffnung reversibel bzw. mehrfach verschließbar ist, um somit ein mehrfaches Nachfüllen des Vorratsbehälters der Beduftungsvorrichtung zu ermöglichen.

Eine Möglichkeit zur Realisierung selbsttätig arbeitender bzw. die Befüllöffnung verschließender Verschlussmittel besteht darin, wenn die Verschlussmittel ein die Befüllöffnung begrenzendes Elastomermaterial umfassen, welches zum Öffnen der Befüllöffnung mittels eines Einfüllstutzens einer Befülleinrichtung, insbesondere bezogen auf eine Einsteckrichtung eines Einfüllstutzens in radialer Richtung, verdrängbar ist und nach Herausziehen des Einfüllstutzens die Befüllöffnung selbsttätig wieder verschließt, d.h. sich wieder ausdehnt. Bevorzugt kann hierfür auch ein Formgedächtnismaterial zum Einsatz kommen.

Erfindungsgemäß umfassen die Verschlussmittel ein selbsttätig verschließbares Rückschlagventil, welches beispielsweise einen entgegen der Schließkraft einer Schließfeder verstellbaren Ventilkörper umfasst, der beispielsweise entgegen der Federkraft der Schließfeder mittels eines Einfüllstutzens während des Befüllvorgangs bzw. zum Befüllvorgang von einer Schließ- in eine Öffnungsposition verstellbar ist.

Wesentlich ist es für eine komfortable Handhabung jedenfalls, dass die Verschlussmittel, unabhängig von der konkreten Realisierungsform, die von der mindestens einen Entleerungsöffnung separate und bevorzugt von dieser beabstandete Befüllöffnung selbsttätig schließen.

Grundsätzlich ist es möglich den Vorratsbehälter als Drucktank auszubilden, in dem die zu verdunstende Flüssigkeit unter Druck steht und unter entsprechender Druckbeaufschlagung dem Vorratsbehälter, in der Art eines Gasfeuerzeugs nachgefüllt wird. Bevorzugt ist jedoch eine Ausführungsform, bei der die zu verdampfende Flüssigkeit in dem Vorratsbehälter drucklos gelagert wird. Im Hinblick auf die konkrete Ausgestaltung des Verdunstungsmaterials gibt es unterschiedliche Möglichkeiten. Mindestvoraussetzung ist, dass das Verdunstungsmaterial eine, bevorzugt große, Verdunstungsfläche anbietet. Hierzu eignet sich insbesondere Filamentmaterial, wie beispielsweise Filz, Wolle und/oder Kunststofffasern. Zusätzlich oder alternativ kann im einfachsten Fall auch ein Papier oder Kartonmaterial als Verdunstungsmaterial eingesetzt werden, oder ein, bevorzugt offenporiges Schwamm- oder Holzmaterial. Bevorzugt handelt es sich um ein von einem Gehäuse der Beduftungsvorrichtung separates, in dem Gehäuse aufgenommenes Material.

Besonders zweckmäßig ist es, wenn die Befüllöffnung an einer anderen Flächenseite des Vorratsbehältnisses angeordnet ist, als die mindestens eine Entleerungsöffnung. Bevorzugt spannen die die unterschiedlichen Öffnungen aufweisenden Flächenseiten einen Winkel, insbesondere von 90° auf oder sind alternativ beispielsweise parallel zueinander angeordnet und dann voneinander über mindestens eine weitere Flächenseite beabstandet. Ganz besonders bevorzugt ist die Befüllvorrichtung von außen zugänglich und hierzu bevorzugt an einer sich bei bestimmungsgemäßer Aufstellung oder Aufhängung der Beduftungsvorrichtung in vertikaler Richtung erstreckender Gehäuseseite, also seitlich angeordnet. Alternativ ist es möglich, die Befüllöffnung an einer (in vertikaler Richtung betrachtet oberen) Oberseite des Vorratsbehältnisses (bei bestimmungsgemäßer Aufstellung bzw. Aufhängung) vorzusehen.

Ganz besonders bevorzugt ist es, wenn die mindestens eine Entleerungsöffnung innerhalb der Beduftungsvorrichtung angeordnet ist, wohingegen die Befüllöffnung bevorzugt von außen zugänglich ist und sich bevorzugt an einer Außenseite bzw. Fläche befindet.

Im Hinblick auf die konkrete Anordnung des Vorratsbehälters gibt es unterschiedliche Möglichkeiten. So ist eine Ausführungsform realisierbar, bei der der Vorratsbehälter bei bestimmungsgemäßer Aufstellung bzw. Aufhängung der Beduftungsvorrichtung in vertikaler Richtung oberhalb der Verdunstungseinrichtung und insbesondere des Verdunstungsmaterials angeordnet ist, sodass zu verdampfende Flüssigkeit schwerkraftbedingt nach unten in die Verdunstungseinrichtung strömen kann. Alternativ ist eine Ausführungsform denkbar, bei der sich bei bestimmungsgemäßer Anordnung der Beduftungsvorrichtung in vertikaler Richtung der Vorratsbehälter unterhalb der Verdunstungseinrichtung befindet, wobei dann bevorzugt die fluidleitende Verbindung zwischen dem Vorratsbehälter und der Verdunstungseinrichtung über Kapillareffekte realisiert ist, insbesondere über in den Vorratsbehälter über die mindestens eine Entleerungsöffnung hineinreichendes Kapillarmaterial, insbesondere Verdunstungsmaterial. Eine weitere Möglichkeit besteht darin, den Vorratsbehälter seitlich benachbart zur Verdunstungseinrichtung anzuordnen, wobei bei einer derartigen Ausführungsform je nach Anordnung der mindestens einen Entleerungsöffnung die zu verdunstende Flüssigkeit schwerkraftbedingt in die Verdunstungseinrichtung strömen kann und/oder durch eine entsprechende Kapillarwirkung von Kapillarmaterial, insbesondere Verdunstungsmaterial.

Um die Beduftungsvorrichtung möglichst kompakt zu gestalten ist in Weiterbildung der Erfindung mit Vorteil vorgesehen, dass der Vorratsbehälter in einem gemeinsamen Gehäuse mit der Verdunstungseinrichtung ausgebildet ist, wobei der Vorratsbehälter selbst Bestandteil des Gehäuses sein kann, was bevorzugt ist. Besonders zweckmäßig ist es, wenn das Gehäuse der Beduftungsvorrichtung quaderförmig ausgestaltet ist. Ganz besonders bevorzugt ist es, wenn der Vorratsbehälter monolithisch mit einem das Verdunstungsmaterial aufnehmenden Gehäuseabschnitt der Verdunstungseinrichtung verbunden bzw. ausgebildet ist. Eine derartige Ausführungsform ist insbesondere dadurch realisierbar, dass das Gehäuse und der Vorratsbehälter, insbesondere als Bestandteil des Gehäuses, als ein- oder mehrteiliges Kunststoffspritzgussteil ausgebildet ist. Im Hinblick auf eine besonders bevorzugte Ausführungsform ist es bevorzugt, wenn das Gehäuse aus zwei Halbschalen besteht, die entsprechend miteinander verbunden werden, im Bereich des Vorratsbehälters bevorzugt im Verbindungsbereich fluiddicht, beispielsweise durch Verschweißen, wie Laserverschweißen oder Ultraschallverschweißen.

Alternativ ist es selbstverständlich möglich, dass der Vorratsbehälter nicht Bestandteil des Gehäuses ist sondern, insbesondere zusammen mit dem Verdunstungsmaterial der Verdunstungseinrichtung in einem gemeinsamen Gehäuse bzw. im Gehäuse aufgenommen ist.

Besonders komfortabel ist es, wenn die Beduftungsvorrichtung einen Ständer umfasst, mit der die Beduftungsvorrichtung auf einer Aufstellfläche abstellbar ist. Ganz besonders bevorzugt ist das gemeinsame Gehäuse der Beduftungsvorrichtung hierzu lösbar in einer Aufnahme des Ständers aufnehmbar. In diesem Fall dient der Ständer im Wesentlichen als Stütze, in die hinein das Gehäuse der Beduftungsvorrichtung samt Vorratsbehälter und Verdunstungseinrichtung einstellbar ist.

Zusätzlich oder alternativ zu dem Vorsehen eines Ständers ist es bevorzugt, wenn die Beduftungsvorrichtung Aufhängemittel, insbesondere in Hakenform aufweist, um das Gehäuse mit samt Vorratsbehälter und Verdunstungseinrichtung aufhängen zu können, beispielsweise an einer Kleiderstange in einem Kleiderschrank oder auch an einer Gardinenstange, etc. Besonders zweckmäßig ist es, wenn die Aufhängemittel lösbar am Gehäuse festlegbar sind, beispielsweise mittels einer Rastverschlussanordnung oder auch einer Gewinde- oder Bajonettverbindung.

Um eine optimale Verdunstung für zu verdunstende Flüssigkeit in den Raum zu ermöglichen ist es bevorzugt, wenn die Verdunstungseinrichtung, insbesondere in deren Gehäuseabschnitt Austrittsöffnungen aufweist, die die Luft von dem Verdunstungsmaterial in den Raum abgebbar ist. Diese können je nach Ausgestaltung dauerhaft offen oder alternativ über entsprechende Funktionsmittel verschließbar ausgestaltet sein.

Ferner führt die Erfindung auch auf ein System, umfassend eine nach dem Konzept der Erfindung ausgebildete Beduftungsvorrichtung sowie einen Fluidtank für Nachfüllflüssigkeit bzw. zu verdunstende Flüssigkeit umfassende Befülleinrichtung zum Befüllen des Vorratsbehälters der Beduftungsvorrichtung durch deren Befüllöffnung hindurch. Besonders bevorzugt ist es, wenn die Befülleinrichtung einen Einfüllstutzen zum Einführen in die Befüllöffnung des Vorratsbehälters aufweist, wobei besonders bevorzugt Einfüllstutzen und Verschlussmittel so aufeinander abgestimmt sind, dass durch den Einführvorgang die Befüllöffnung öffenbar und durch Herausziehen selbsttätig verschließbar ist. Besonders zweckmäßig ist es, wenn die Befülleinrichtung eine, insbesondere manuelle Pumpeinrichtung aufweist, um, insbesondere durch mehrere Pumphübe, zu verdampfende Flüssigkeit aus dem Fluidtank der Befülleinrichtung in den Vorratsbehälter der Beduftungseinrichtung zu fördern.

Weitere Vorteile, Merkmale und Einzelheiten der Erfindung ergeben sich aus der nachfolgenden Beschreibung bevorzugter Ausführungsbeispiele sowie anhand der Zeichnungen.

Diese zeigen in:
- Fig. 1:: ein erstes Ausführungsbeispiel einer nach dem Konzept der Erfindung ausgebildeten Beduftungsvorrichtung mit fakultativen Aufhängmitteln und Ständer,
- Fig. 2a - 2c.: unterschiedliche Ansichten einer alternativen Ausführungsform einer nach dem Konzept der Erfindung ausgebildeten Beduftungsvorrichtung.

In den Figuren sind gleiche Elemente und Elemente mit der gleichen Funktion mit den gleichen Bezugszeichen gekennzeichnet.

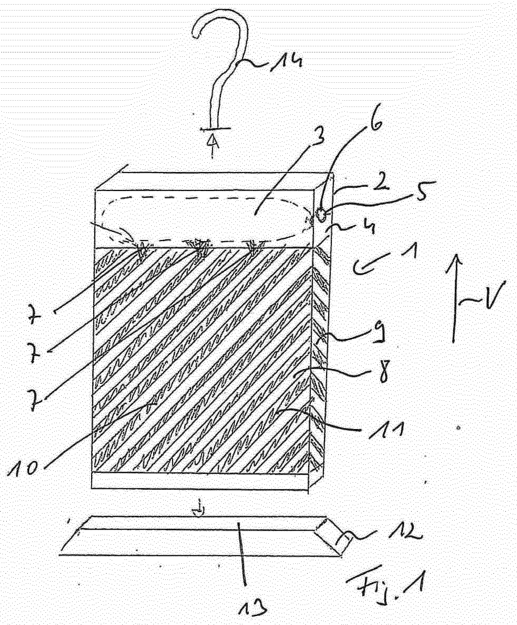

Fig. 1 zeigt ein erstes Ausführungsbeispiel einer nach dem Konzept der Erfindung ausgebildeten Beduftungsvorrichtung 1. Diese umfasst ein Gehäuse 2, welches vorliegend beispielhaft eine Quaderform aufweist und in dem ein Vorratsbehälter 3 (Tank) zur Aufnahme von zu verdunstender Flüssigkeit aufgenommen ist. An einer Gehäuseaußenseite, vorliegend einer in vertikaler Richtung orientierten Gehäuseseitenfläche 4 ist eine Befüllöffnung 5 angeordnet, über die zu verdunstende Flüssigkeit in den Vorratsbehälter 3 manuell von außen einfüllbar ist. Der Befüllvorrichtung 5 sind Verschlussmittel 6 zugeordnet, vorliegend in der Form von Elastomermaterial, welches die Befüllöffnung 5 begrenzt und selbsttätig verschließt. Zum Öffnen der Befüllöffnung 5 wird bevorzugt ein Einfüllstutzen eingeführt, der die Befüllöffnung 5 vergrößert bzw. das Elastomermaterial nach radial außen verdrängt, wobei nach Herausziehen des Einfüllstutzens die Öffnung selbsttätig verschlossen wird. Alternative Verschlussmittel sind realisierbar. Bevorzugt wird die Beduftungsvorrichtung 1 zum Befüllen des Vorratsbehältnisses 3 verglichen mit der Darstellung um 90° gedreht, also derart, dass die Befüllöffnung 5 in vertikaler Richtung nach oben weist.

Aus dem Vorratsbehälter 3 gelangt zu verdunstende Flüssigkeit über von der Befüllöffnung 5 separate und von dieser beanstandete, in dem konkreten Ausführungsbeispiel lediglich beispielhaft drei Entleerungsöffnungen 7 in eine Verdunstungseinrichtung 8, die von einem Gehäuseabschnitt 9 des Gehäuses 2 begrenzt ist. In dem Gehäuseabschnitt 9 befinden sich Austrittsöffnungen 10, durch die hindurch Duftstoffe in den Raum hinein diffundieren können, ausgehend von einem Verdunstungsmaterial 11 innerhalb der Verdunstungseinrichtung 8 bzw. des Gehäuseabschnitts 9, welches eine entsprechend große Verdunstungsoberfläche bereitstellt, beispielsweise indem das Verdunstungsmaterial 11 aus einem Filzmaterial od.dgl. Oberflächenmaterial besteht.

Je nach Ausgestaltung der Beduftungsvorrichtung 1 kann die zu verduftende Flüssigkeit aus dem Vorratsbehälters 3 unmittelbar aus den Entleerungsöffnungen 7, vorliegend schwerkraftbedingt, in das Verdunstungsmaterial 11 fließen bzw. tropfen - denkbar ist es, fluidleitendes Material, beispielsweise aufgrund einer Kapillarwirkung als flüssigkeitsleitende Verbindung durch die Entleerungsöffnungen 7 hindurch vorzusehen, insbesondere wenn der Vorratsbehälter 3 wie vorliegend nicht in vertikaler Richtung V oberhalb der Verdunstungseinrichtung 8 angeordnet ist sondern unterhalb oder seitlich.

Wie aus Fig. 1 zu erkennen ist, umfasst die Beduftungsvorrichtung 1 bevorzugt einen Ständer 12 zum Aufstellen auf einer Aufstellfläche, wobei das Gehäuse 2 lösbar in einer Aufnahme 13 des Ständers 12 aufnehmbar ist. Zusätzlich oder alternativ können bevorzugt, wie vorliegend, hakenförmige Aufhängmittel 14 realisiert werden, die bevorzugt lösbar mit dem Gehäuse 2 verbindbar bzw. verbunden sind, um das Gehäuse 2, beinhaltend den Vorratsbehälters 3 und das Verdunstungsmaterial 11, beispielsweise an einer Kleiderstange aufhängen zu können.

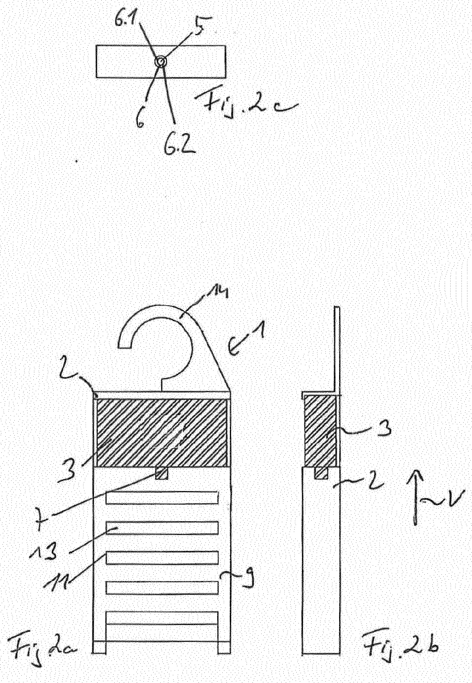

Fig. 2a bis 2b zeigen mehrere Ansichten einer alternativen, nach dem Konzept der Erfindung ausgebildeten Verdunstungseinrichtung 8. Hier sind beispielhaft die Aufhängmittel 14 Bestandteil eines gemeinsamen Gehäuses 2, in welchem der Vorratsbehälter 3 aufgenommen ist. Dieser ist auch in dem gezeigten Ausführungsbeispiel lediglich beispielhaft oberhalb der Verdunstungseinrichtung 8 angeordnet, durch deren Austrittsöffnungen 10 im Gehäuseabschnitt 9 Duftstoffe austreten können. Innerhalb der Verdunstungseinrichtung 8 befindet sich nicht sichtbares Verdunstungsmaterial 11. Über eine Entleerungsöffnung 7 kann zu verdunstende (Duft-)Flüssigkeit aus dem Vorratsbehälter 3 in das Verdunstungsmaterial 11 gelangen.

Aus der in Fig. 2c gezeigten Draufsicht ist die Befüllöffnung 5 zu erkennen mit ihren Verschlussmitteln 6 zum selbsttätigen Verschließen der Befüllöffnung 5 nach einem Befüllvorgang angeordnet. Die Verschlussmittel 6 umfassen ein erfindungsgemäßes, selbsttätig schließendes Rückschlagventil 6.1. Dieses kann beispielsweise einen entgegen der Schließkraft einer Schließfeder verstellbaren Ventilkörper 6.2 umfassen, der beispielsweise entgegen der Federkraft einer in der Seitenansicht der Fig. 2c nicht erkennbaren Schließfeder mittels eines Einfüllstutzens während des Befüllvorgangs bzw. zum Befüllvorgang von einer Schließ- in eine Öffnungsposition verstellbar ist.

Bei sämtlichen gezeigten Ausführungsbeispielen ist die Befüllöffnung 5 von außen zugänglich und damit an einer anderen Flächenseite als die mindestens eine Entleerungsöffnung 7.

### Bezugszeichen

- 1: Beduftungsvorrichtung
- 2: Gehäuse
- 3: Vorratsbehälter
- 4: Gehäuseseite
- 5: Befüllöffnung
- 6: Verschlussmittel
- 6.1: Rückschlagventil
- 6.2: Ventilkörper
- 7: Entleerungsöffnung(en)
- 8: Verdunstungseinrichtung
- 9: Gehäuseabschnitt der Verdunstungseinrichtung
- 10: Austrittsöffnung(en) der Verdunstungseinrichtung
- 11: Verdunstungsmaterial
- 12: Ständer
- 13: Aufnahme
- 14: Aufhängmittel

- V: Vertikalrichtung

## Patentansprüche

1. Beduftungsvorrichtung (1) zum Abgeben eines Duftes in einen Raum, mit einem Vorratsbehälter (3) für zu verdunstende Flüssigkeit sowie mit einem mit dem Vorratsbehälter (3) über mindestens eine Entleerungsöffnung (7) in fluidleitender Verbindung stehenden, eine Verdunstungsoberfläche anbietenden Verdunstungsmaterial (11), insbesondere Filz, Karton, Holz oder Schwammmaterial umfassenden Verdunstungseinrichtung (8), wobei der Vorratsbehälter (3) eine von der mindestens einen Entleerungsöffnung (7) separate Befüllöffnung (5) zum individuellen Befüllen aufweist, die über dieser zugeordnete Verschlussmittel (6), bevorzugt selbsttätig, nach einem Befüllvorgang, bevorzugt reversibel, verschließbar ist
**dadurch gekennzeichnet,**
**dass** die Verschlussmittel (6) ein selbsttätig schließendes Rückschlagventil (6.1) umfassen.

2. Beduftungsvorrichtung nach Anspruch 1,
**dadurch gekennzeichnet,**
**dass** die Verschlussmittel (6) die Befüllöffnung (5) begrenzendes Elstomermaterial umfassen, welches zum Öffnen der Befüllöffnung (5) mittels eines Einfüllstutzens einer Befülleinrichtung verdängbar und nach Herausziehen des Einfüllstutzens die Befüllöffnung (5) selbsttätig verschließend ausgebildet ist.

3. Beduftungsvorrichtung nach einem der vorhergehenden Ansprüche,
**dadurch gekennzeichnet,**
**dass** die Befüllöffnung (5) an einer anderen Flächenseite des Vorratsbehältnisses (3) angeordnet ist als die Entleerungsöffnung (7).

4. Beduftungsvorrichtung nach einem der vorhergehenden Ansprüche,
**dadurch gekennzeichnet,**
**dass** der Vorratsbehälter (3) in einem gemeinsamen Gehäuse (2) mit der Verdunstungseinrichtung (8) ausgebildet ist, wobei bevorzugt der Vorratsbehälter (3) monolithisch mit einem das Verdunstungsmaterial (11) aufnehmenden Gehäuseabschnitt (9) der Verdunstungseinrichtung (8) verbunden ist.

5. Beduftungsvorrichtung nach Anspruch 4,
**gekennzeichnet**
**durch** einen Ständer (12) zur insbesondere lösbaren Aufnahme (13) des gemeinsamen Gehäuses (2) und zum Abstellen des Gehäuses (2) auf einer Aufstellfläche.

6. Beduftungsvorrichtung nach einem der Ansprüche 4 oder 5,
**gekennzeichnet**
**durch**, bevorzugt lösbar am Gehäuse (2) festlegbare, besonders bevorzugt hakenförmige, Aufhängmittel (14) zum Aufhängen des Gehäuses (2), insbesondere an einer Kleiderstange.

7. Beduftungsvorrichtung nach einem der vorhergehenden Ansprüche,
**dadurch gekennzeichnet,**
**dass** die Verdunstungseinrichtung (8) Austrittsöffnungen (10) aufweist, durch die Duft von dem Verdunstungsmaterial (11) in den Raum abgebbar ist.

8. System, umfassend eine Beduftungsvorrichtung (1) nach einem der vorhergehenden Ansprüche sowie einen, bevorzugt eine Pumpeinrichtung umfassende, einen Fluiddtank umfassende Befülleinrichtung zum Befüllen des Vorratsbehälters (3) der Beduftungsvorrichtung (1), bevorzugt umfassend einen Einfüllstützen zum Einführen in die Befüllöffnung (5) des Vorratsbehälters (3).
